# EUROPEAN PATENT APPLICATION

(11) **EP 4 725 388 A1**
(43) Date of publication of application: **15.04.2026**
(21) Application number: 23940718.2
(22) Date of filing: 08.06.2023
(51) Int. Cl.: A61B 1/04

(54) **IMAGING MODULE, ENDOSCOPE, AND IMAGING MODULE MANUFACTURING METHOD**

(71) Applicant: Olympus Medical Systems Corp., Tokyo 192-8507 (JP)
(72) Inventor: KOBAYASHI, Keiichi, Hachioji-shi, Tokyo 192-8507 (JP); KODAMA, Daichi, Hachioji-shi, Tokyo 192-8507 (JP); YAMADA, Junya, Hachioji-shi, Tokyo 192-8507 (JP)
(74) Representative: Gunzelmann, Rainer
(86) International application number: PCT/JP2023/021361
(87) International publication number: WO 2024/252620

(57) **Abstract**

An image pickup module includes a wiring board including a first principal surface and a second principal surface, a land and a draw-out wiring being disposed on the first principal surface, the wiring board including a through hole, a through wiring layer in electrical communication with the draw-out wiring being disposed on an inner surface of the through hole; a resin disposed in the through hole and sealing an opening on a side of the first principal surface; a bonding member bonded to the land; and a camera unit bonded to the bonding members.

## Description

### BACKGROUND OF THE INVENTION

### 1. TECHNICAL FIELD

The present disclosure relates to an image pickup module in which a camera unit is disposed, an endoscope including the image pickup module in which the camera unit is disposed, and a method for manufacturing the image pickup module in which the camera unit is disposed.

### 2. DESCRIPTION OF THE RELATED ART

In recent years, three-dimensional circuit devices, such as molded interconnect devices (MIDs), have been used to miniaturize electronic devices and more sophisticated.

The publication of Japanese Patent Application Laid-Open Publication No. 2017-23234 discloses a camera unit of an endoscope using an irregularly-shaped circuit substrate, which is a three-dimensional circuit device. The camera unit includes an image pickup device, a flat wiring board (planar wiring board) on which an electronic component is mounted, and an irregularly-shaped circuit substrate (three-dimensional wiring board). A plurality of cables is bonded to each of a plurality of side surfaces of the irregularly-shaped circuit substrate.

International Publication No. 2021/181530 discloses an image pickup module in which a camera unit is mounted in a cavity of a molded interconnect device. External electrodes of the camera unit are connected to signal cables via through wirings of through holes that penetrate through a bottom surface of a cavity to lead to a back surface.

When the camera unit is solder-bonded to the three-dimensional circuit device, if the solder flows out into the through holes, not only solder-bond reliability may be reduced, but also the camera unit may move from a predetermined position. If an optical axis of the camera unit shifts from the predetermined position, a desired field of view may not be obtained, and the performance of an image pickup apparatus may be reduced.

### PRIOR ART DOCUMENT

### PATENT DOCUMENT

Patent document 1: The publication of Japanese Patent Application Laid-Open Publication No. 2017-23234
Patent document 2: International Publication No. 2021/181530

An objective of the present disclosure is to provide a high-performance image pickup module in which a camera unit is precisely bonded, an endoscope including the high-performance image pickup module in which the camera unit is precisely bonded, and a method for manufacturing the image pickup module in which the camera unit is precisely bonded.

### SUMMARY OF THE INVENTION

An image pickup module of an aspect of the present disclosure includes a wiring board including a first principal surface and a second principal surface on an opposite side of the first principal surface, a plurality of lands and a plurality of draw-out wirings extended respectively from the plurality of lands being disposed on the first principal surface, the wiring board including a plurality of through holes penetrating through the first principal surface and the second principal surface, a plurality of through wiring layers being disposed on respective inner surfaces of the plurality of through holes, the plurality of through wiring layers being in electrical communication with the plurality of draw-out wirings, respectively; a plurality of resins disposed respectively in the plurality of through holes and each sealing an opening on a side of the first principal surface; a plurality of bonding members bonded respectively to the plurality of lands; and a camera unit bonded to the plurality of bonding members.

An endoscope of an aspect of the present disclosure includes an insertion portion including a distal end portion in which an image pickup module is disposed. The image pickup module includes a wiring board including a first principal surface and a second principal surface on an opposite side of the first principal surface, a plurality of lands and a plurality of draw-out wirings extended respectively from the plurality of lands being disposed on the first principal surface, the wiring board including a plurality of through holes penetrating through the first principal surface and the second principal surface, a plurality of through wiring layers being disposed on respective inner surfaces of the plurality of through holes, the plurality of through wiring layers being in electrical communication with the plurality of draw-out wirings, respectively; a plurality of resins disposed respectively in the plurality of through holes and each sealing an opening on a side of the first principal surface; a plurality of bonding members bonded respectively to the plurality of lands; and a camera unit bonded to the plurality of bonding members.

A method for manufacturing an image pickup module of an aspect of the present disclosure includes steps of: manufacturing a wiring board including a first principal surface and a second principal surface on an opposite side of the first principal surface, a plurality of lands and a plurality of draw-out wirings extended respectively from the plurality of lands being disposed on the first principal surface, the wiring board including a plurality of through holes penetrating through the first principal surface and the second principal surface, a plurality of through wiring layers being disposed on respective inner surfaces of the plurality of through holes, the plurality of through wiring layers being in electrical communication with the plurality of draw-out wirings, respectively; disposing a plurality of resins in the plurality of through holes, respectively, to thereby sealing an opening of each of the plurality of through holes on a side of the first principal surface; and bonding a plurality of external electrodes of a camera unit to the plurality of lands, respectively, by using a bonding member.

According to an embodiment of the present disclosure, it is possible to provide a high-performance image pickup module in which a camera unit is precisely bonded, an endoscope including the high-performance image pickup module in which the camera unit is precisely bonded, and a method for manufacturing the high-performance image pickup module in which the camera unit is precisely bonded.

### BRIEF DESCRIPTION OF THE DRAWINGS

Fig. 1 is a perspective view of an image pickup module of a first embodiment.
Fig. 2 is a perspective view of the image pickup module of the first embodiment.
Fig. 3 is a cross-sectional view along a III-III line of Fig. 1.
Fig. 4 is a partial enlarged view of Fig. 3.
Fig. 5 is a flowchart of a method for manufacturing the image pickup module of the first embodiment.
Fig. 6 is a perspective view of a base of a wiring board of the image pickup module of the first embodiment.
Fig. 7 is a partial cross-sectional view to describe the method for manufacturing the image pickup module of the first embodiment.
Fig. 8 is a partial cross-sectional view to describe the method for manufacturing the image pickup module of the first embodiment.
Fig. 9 is a partial cross-sectional view to describe the method for manufacturing the image pickup module of the first embodiment.
Fig. 10 is a partial cross-sectional view to describe the method for manufacturing the image pickup module of the first embodiment.
Fig. 11 is a perspective view to describe the method for manufacturing the image pickup module of the first embodiment.
Fig. 12 is a bottom view of a camera unit of the image pickup module of the first embodiment.
Fig. 13 is a bottom view of a cavity of the wiring board of the image pickup module of the first embodiment.
Fig. 14 is a bottom view of the cavity of the wiring board of the image pickup module of the first embodiment.
Fig. 15 is a perspective view of an endoscope of a second embodiment.
Fig. 16 is a perspective view of a main portion of a distal end member of the endoscope of the second embodiment.
Fig. 17 is a cross-sectional view along an XVII-XVII line of Fig. 16.

### DETAILED DESCRIPTION

The following describes of embodiments of the present disclosure with reference to the drawings.

Note that the drawings based on the embodiments are schematic. The relationship between the thickness and width of each part and the ratio of the thickness of each part differ from the actual ones. The drawings also contain parts in which dimensional relationships and ratios are different from each other.

### <First Embodiment>

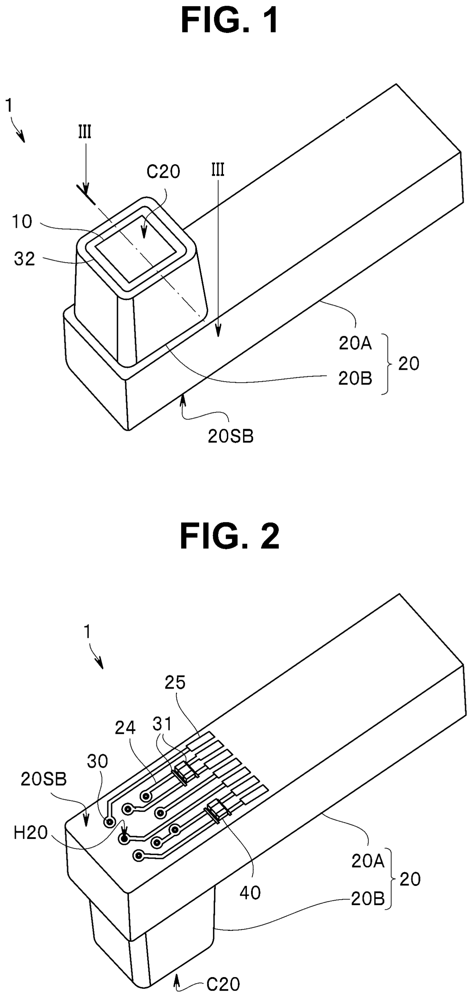

Figs. 1 to 4 show an image pickup module 1 of the present embodiment. The image pickup module 1 includes a wiring board 20 and a camera unit 10. The wiring board 20 is a three-dimensional wiring board, which is a molded interconnect device (MID), as mentioned below.

The wiring board 20 is a 3D (three-dimensional) molded interconnect device in which a plurality of conductor patterns and the like are disposed on an injection-molded three-dimensional substrate. Unlike conventional flat wiring boards, use of the wiring board 20 allows the shape to have a function, and furthermore, a conductor pattern can be formed on an inclined surface, a vertical surface, a curved surface, in a through hole, and the like.

The wiring board 20 includes an assembly member 20A and a protrusion 20B protruding from the assembly member 20A. The protrusion 20B, which is surrounded by framed walls, configures a cavity C20 which is a bottomed hole and in which the camera unit 10 is to be housed. A gap between the camera unit 10 housed in a cavity C20 and an inner wall surface 20SS of the cavity C20 is filled with a sealing resin 32. Hereinafter, a bottom surface of the cavity C20 will be referred to as a first principal surface 20SA, and a surface on the opposite side of the first principal surface 20SA will be referred to as a second principal surface 20SB.

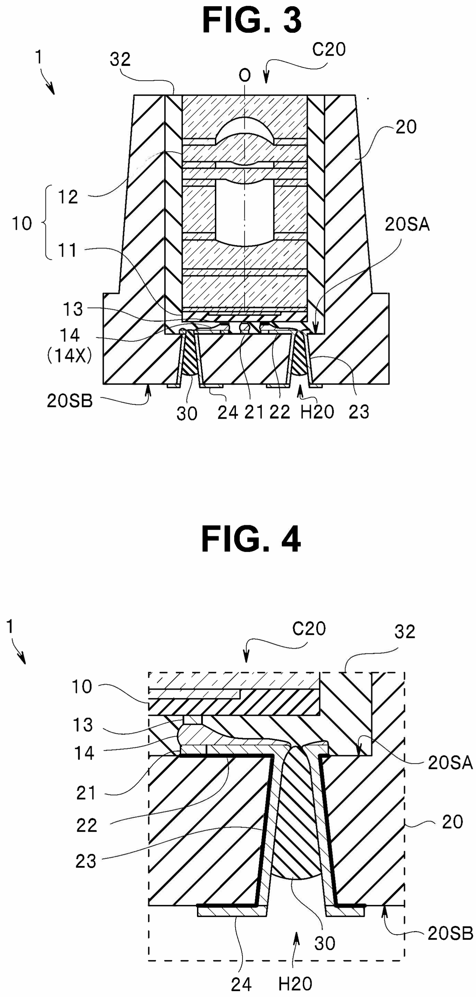

As shown in Figs. 3 and 4, the camera unit 10 includes an imager 11 and an optical element 12. The optical element 12 includes a plurality of lenses and the like. The imager 11 is an image pickup device such as a CCD or CMOS that converts an object image condensed by the optical element 12 into an electrical signal.

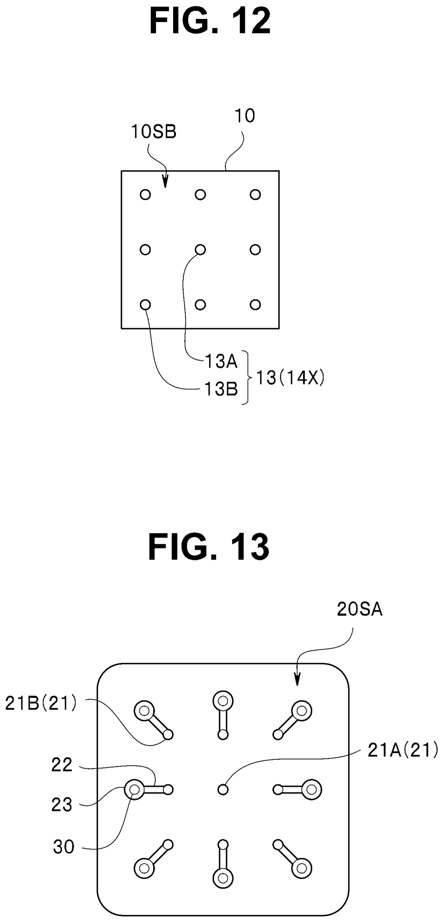

The camera unit 10 (imager 11) includes, on the bottom surface 10SB, a plurality of external electrodes 13 that transmit and receive electrical signals. On the external electrodes 13, solder bumps 14X (see Fig. 12) are respectively disposed, thus forming a ball grid array. The camera unit 10 is bonded to the wiring board 20 by the solder 14, which is a bonding member formed by melting the solder bumps 14X.

The wiring board 20 includes a plurality of through holes H20 that penetrate through the first principal surface 20SA and the second principal surface 20SB. A through wiring layer 23 is disposed on the inner surface of the through holes H20. As mentioned below, a resin 30 is disposed in the through holes H20. On the second principal surface 20SB, a wiring pattern 24 extended from the through wiring layer 23, and a pad 25 provided at an end portion of the wiring pattern 24 are disposed.

A plurality of solder resist patterns 31 are disposed on the wiring pattern 24 on the second principal surface 20SB. An electronic component, for example, a chip capacitor 40, is surface-mounted on two wiring patterns 24 between two solder resist patterns 31. The solder resist patterns 31 are disposed for mounting an electronic component on the two wiring patterns 24, which are placed in parallel. Although not shown in the figure, a signal cable is bonded to the pad 25.

On the first principal surface 20SA, a land 21 and a draw-out wiring 22 extended from the land 21 are disposed. An end portion of the draw-out wiring 22 is connected to the through wiring layer 23.

As mentioned below, the land 21, the draw-out wiring 22, the through wiring layer 23, the wiring pattern 24, and the pad 25 are conductor layers that are deposited simultaneously, and their boundaries are not clear.

The external electrode 13 of the camera unit 10 is bonded to the land 21 of the wiring board 20 by the solder 14 resulting from the melting of the solder bump 14X. The solder 14 melted during bonding spreads from the land 21 to the draw-out wiring 22.

An inner diameter of the through hole H20 is greater than twice a thickness of the through wiring layer 23. The through hole H20 is not blocked by the through wiring layer 23, and the first principal surface 20SA includes an opening. However, this opening is blocked by the resin 30. Therefore, the molten solder 14 does not flow into the through hole.

As mentioned below, the camera unit 10 which is not fixed when the camera unit 10 is bonded to the wiring board 20 may move in a direction in which the solder 14 flows out when the solder bump 14X melts. If the solder 14 flows into the through hole H20, the bonding between the external electrode 13 and the land 21 may become weak or the camera unit 10 may be bonded tilted.

In the image pickup module 1, since the opening of the through hole H20 in the first principal surface 20SA is sealed by the resin 30, the camera unit 10 is bonded precisely to a desired position on the wiring board 20. Therefore, the image pickup module 1 has high performance.

### <Method for Manufacturing Image Pickup Module>

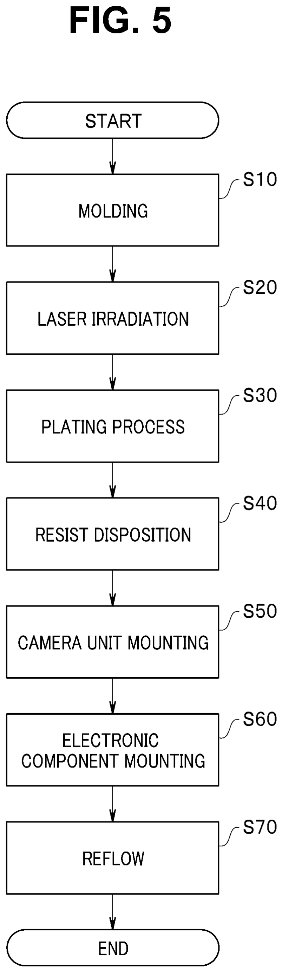

A method for manufacturing the image pickup module will be described below along the flowchart in Fig. 5.

### <Step S10> Molding (Molded Substrate Fabrication Process)

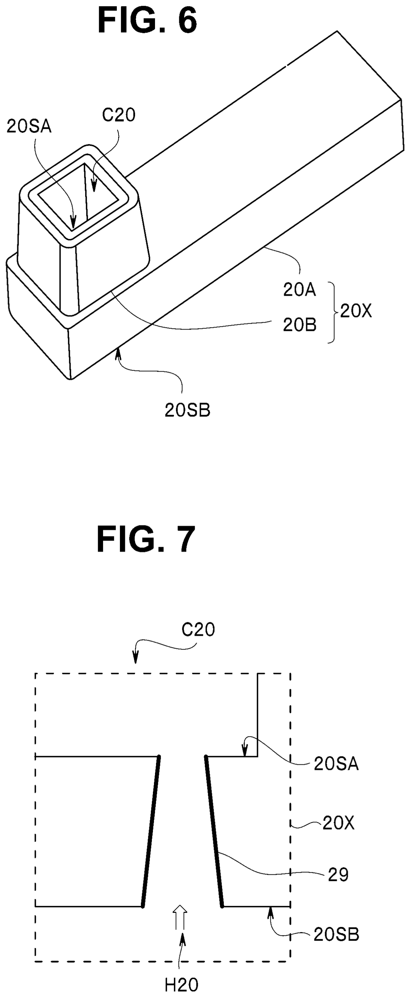

An MID resin is poured in a mold (not shown) including a recess in the shape of the wiring board 20. Injection molding is used to fabricate a molded substrate 20X, as shown in Fig. 6. The MID resin is doped with a precursor such as a non-conductive metal complex that becomes a plating catalyst by being irradiated with light.

### <Step S20> Laser Irradiation (Through Hole Formation Process and Active Layer Formation Process)

As shown in Fig. 7, the second principal surface 20SB of the molded substrate 20X is irradiated with high-output laser, thereby forming the through hole H20 extending from the second principal surface 20SB to the first principal surface 20SA. The through hole H20 has a larger opening on the second principal surface 20SB than an opening on the first principal surface 20SA. In other words, an inner dimension of the through hole H20 increases from the first principal surface 20SA to the second principal surface 20SB. A catalyst layer 29 activated is formed on the wall surface of the through hole H20.

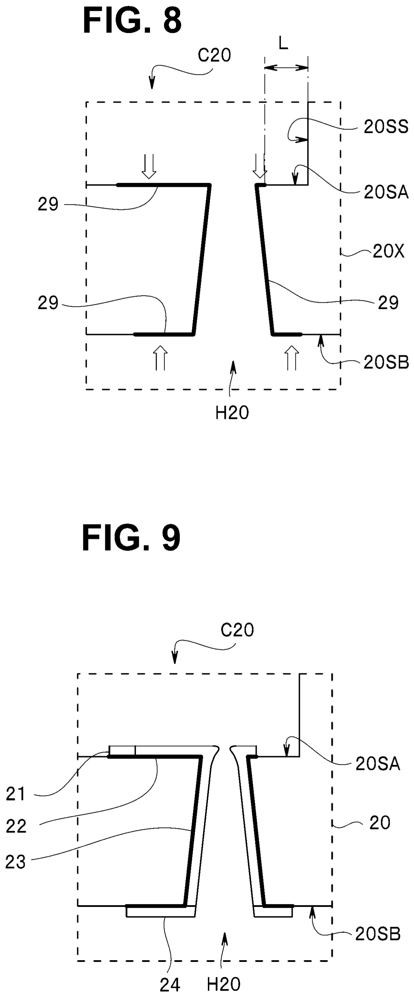

As shown in Fig. 8, the first principal surface 20SA and the second principal surface 20SB are pattern-irradiated with the laser in order to dispose the land 21, the draw-out wiring 22, the wiring pattern 24, and the pad 25. By the laser irradiation, the catalyst layer 29 with catalytic activity for electroless plating is formed.

It is not easy to irradiate with laser an outer circumference region of the first principal surface 20SA, which is the bottom surface of the cavity C20. If the wall surface 20SS of the cavity C20 is irradiated with the laser, a catalyst layer is formed on the wall surface 20SS and a plating film is deposited thereon. Therefore, as shown in Fig. 8, the end portion on the outer circumference side of the draw-out wiring 22 may extend by a length greater than a predetermined distance L from the inner wall surface 20SS of the cavity C20. For example, the predetermined distance L may be greater than 0.1 mm, and particularly greater than 0.2 mm.

### <Step S30> Plating Process

As shown in Fig. 9, by performing an electroless plating process, the molded substrate 20X becomes the wiring board 20 in which the conductor pattern is disposed on the first principal surface 20SA, the second principal surface 20SB, and the through hole H20. The conductor includes, for example, a copper layer on which a barrier layer made of nickel/gold is disposed.

For example, the through wiring layer 23 has an opening diameter of 0.05 mm on the first principal surface 20SA and an opening diameter of 0.15 mm on the second principal surface 20SB.

### <Step S40> Resist (Resin) Disposition

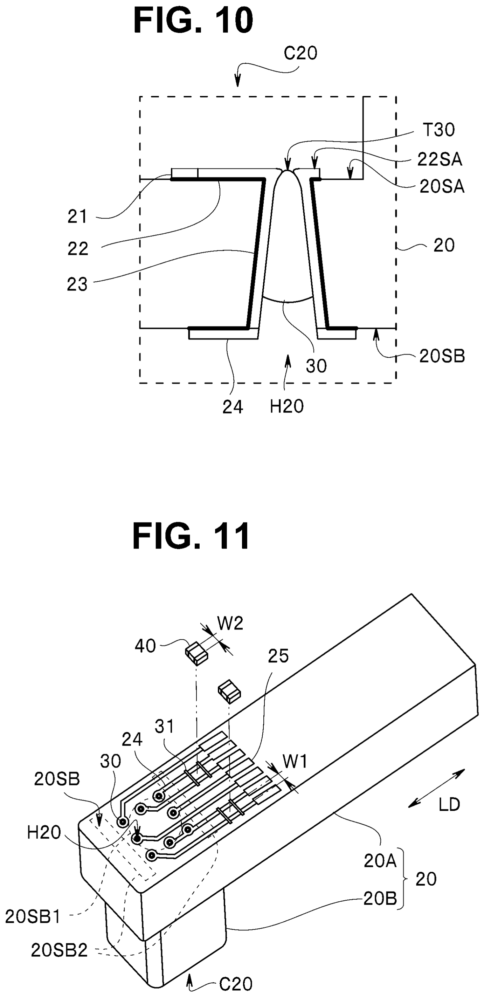

As shown in Fig. 10, a dispenser is used to dispose the resin 30 into the through hole H20 from the second principal surface 20SB side. The resin 30 may be a solder resist. The solder resist is a resin that is solder resistant, has a large interfacial tension with the solder 14, and prevents solder adhesion to unnecessary parts. For example, the base material of the solder resist is a thermosetting epoxy resin.

An end surface T30 of the resin 30 on the first principal surface 20SA side may be located between a surface 22SA of the draw-out wiring 22 and the first principal surface 20SA. The resin 30 only has to seal at least the opening of the through hole H20 on the first principal surface 20SA side. In other words, the resin 30 does not have to completely fill the through hole H20.

It is noted that the through wiring layer 23 is thick in the vicinity of the first principal surface 20SA, where the through hole H20 has a smaller opening. Therefore, an inner surface of the through wiring layer 23 has a gently protruding step in the vicinity of the first principal surface 20SA. This is considered to be because the deposition speed is faster in the region where the cross-sectional area of the through hole H20 is smaller than in other regions due to the faster diffusion speed of metallic ions during plating deposition.

Due to the presence of the step in the vicinity of the first principal surface 20SA, the resin 30 poured in the through hole H20 does not spread to the surface 22SA of the draw-out wiring 22.

As shown in Fig. 11, when the resin 30 is disposed on the second principal surface 20SB, the solder resist patterns 31 are also simultaneously disposed using the same dispenser. When the same resin (solder resist) as the solder resist patterns 31 is used as the resin 30, the resin 30 and the solder resist patterns 31 can be disposed in a single process, resulting in high productivity.

### <Step S50> Camera Unit Mounting

As shown in Fig. 12, the plurality of external electrodes 13 disposed on the bottom surface 10SB of the camera unit 10 include a plurality of second external electrodes 13B placed at four times symmetrical positions around the first external electrode 13A which is located at the optical axis O. In other words, the plurality of second external electrodes 13B are placed at positions where the electrodes are superimposed when rotated 90 degrees with respect to the center. On the plurality of external electrodes 13, the solder bumps 14X are respectively disposed.

As shown in Fig. 13, on the first principal surface 20SA of the wiring board 20, the land 21 to be bonded to the external electrode 13 of the camera unit 10 by the solder 14 is disposed. The land 21 includes a first land 21A to be connected to the first external electrode 13A of the camera unit 10 and a second land 21B to be connected to the second external electrode 13B.

From each of a plurality of second lands 21B, a draw-out wiring 22 is radially extended. The end portion of the draw-out wiring 22 is extended to the through wiring layer 23 disposed in the through wiring 30.

It is noted that the plurality of second external electrodes 13B, the plurality of second lands 21B, and a plurality of draw-out wirings 22 may be placed at N symmetrical positions (N is a natural number equal to or greater than 2) around the first external electrode 13A (first land 21A).

The camera unit 10 is housed and temporarily fixed in the cavity C20 in a state where each of the plurality of external electrodes 13 is aligned with each of a plurality of lands 21.

### <Step S60> Electronic Component Mounting

An electronic component, for example, the chip capacitor 40, is temporarily fixed between two solder resist patterns 31 on the second principal surface 20SB of the wiring board 20. A terminal of the chip capacitor 40 has a solder plating film, for example. Solder paste may be applied at a bonding position of the wiring pattern 24. It is noted that an interval W1 between the two wiring patterns 24 placed in parallel as shown in Fig. 11 is substantially the same as an interval W2 between terminals of the chip capacitor 40 having two terminals.

It is noted that the electronic component may also be mounted in a region 20SB1 (see Fig. 11) of the second principal surface 20SB, which is on the opposite side of the bottom center region of the protrusion 20B. However, the thickness of the region 20SB1 (e.g., 0.5 mm) is smaller than thicknesses of other regions. Since the region 20SB1 is subject to high deformation due to, for example, thermal expansion/shrinkage, the electronic component mounted on the region 20SB1 may be damaged or have bonding failure. Therefore, the electronic component mounted on the region 20SB1 may have a large bonding area.

On the other hand, the electronic component may also be mounted on a region 20SB2 (see Fig. 11) of the second principal surface 20SB, which is on the opposite side of a wall of the protrusion 20B. The thickness of the region 20SB2 is greater than thicknesses of other regions. Because of having low thermal deformation, the region 20SB2 is suitable for mounting a small electronic component.

It is noted that the thermal deformation of the wiring board 20 is large in a long axis direction LD and small in a direction orthogonal to the long axis direction LD. Therefore, two terminals of the electronic component may be placed in the direction orthogonal to the long axis direction LD.

### <Step S70> Reflow

For example, a reflow furnace is used to heat the image pickup module 1 to a temperature at which the solder 14 melts. When the image pickup module 1 returns to a room temperature, the external electrode 13 of the camera unit 10 is solder-bonded to the land 21 of the wiring board 20. The chip capacitor 40 is also solder-bonded to the wiring pattern 24.

After the reflow process, the sealing resin 32 is poured in and cured between the cavity C20 and the camera unit 10, thereby completing the image pickup module 1.

In the reflow process, the camera unit 10 may move in the direction in which the solder 14 flows out when the solder 14 melts. If the solder 14 flows into the through hole H20, the bonding between the external electrode 13 and the land 21 may become weak or the camera unit 10 may be bonded tilted.

In the image pickup module 1, the plurality of draw-out wirings 22 onto which the solder 14 flows out have the same width and length. In other words, after the reflow, the plurality of draw-out wirings 22 onto which the solder 14 has flowed out have the same area.

In the image pickup module 1, the opening of the through hole H20 is sealed by the resin 30. The solder 14 flows out along the plurality of draw-out wirings 22, which are placed at N symmetrical positions (N is a natural number equal to or greater than 2). Thus, the camera unit 10 is automatically bonded to the desired position precisely by the self-alignment effect during the reflow process. According to the present manufacturing method, the image pickup module 1 having high-performance can be manufactured.

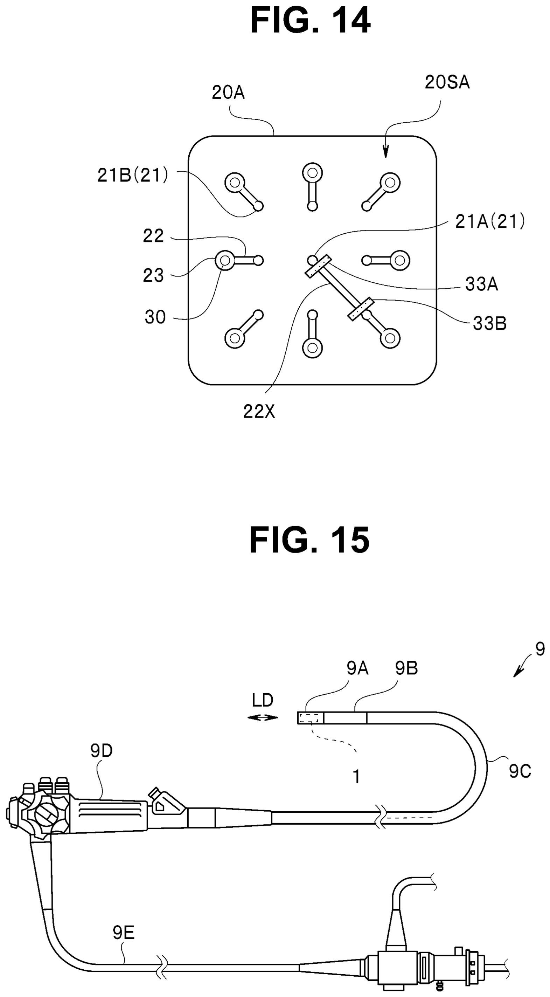

It is noted that, in the wiring board 20 shown in Fig. 14, a dummy draw-out wiring 22X is extended from the first land 21A to one of the second lands 21B. Solder resist patterns 33 (33A, 33B) are disposed on both end portions of the dummy draw-out wiring 22X, respectively. The solder resist patterns 33 may cover an entire surface of the dummy draw-out wiring 22X. The solder resist pattern 33 prevents the solder 14 from flowing out onto the dummy draw-out wiring 22X.

Also in the wiring board 20 shown in Fig. 14, regions where the solder 14 spreads onto the plurality of draw-out wirings 22 are isotropic around the first land 21A, and areas of regions where the solder 14 spreads onto the plurality of draw-out wirings 22 are substantially the same.

### <Second Embodiment>

As shown in Fig. 15, an endoscope 9 of the present embodiment includes a distal end portion 9A in which the image pickup module 1 is disposed, a bending portion 9B which is bendable and provided continuously with a proximal end of the distal end portion 9A, and a flexible portion 9C which is elongated and provided continuously with a proximal end of the bending portion 9B. The bending portion 9B is bent by an operation to an operation portion 9D by a user. The distal end portion 9A, the bending portion 9B, and the flexible portion 9C serve as an insertion portion to be inserted into a body. A universal cord 9E extended from the operation portion 9D is connected to a processor or the like (not shown). It is noted that Fig. 15 shows the long axis direction LD of the distal end portion 9A which is elongated.

The endoscope 9 of the present embodiment is of the so-called side-viewing type, in which an image is picked up in a side direction during insertion.

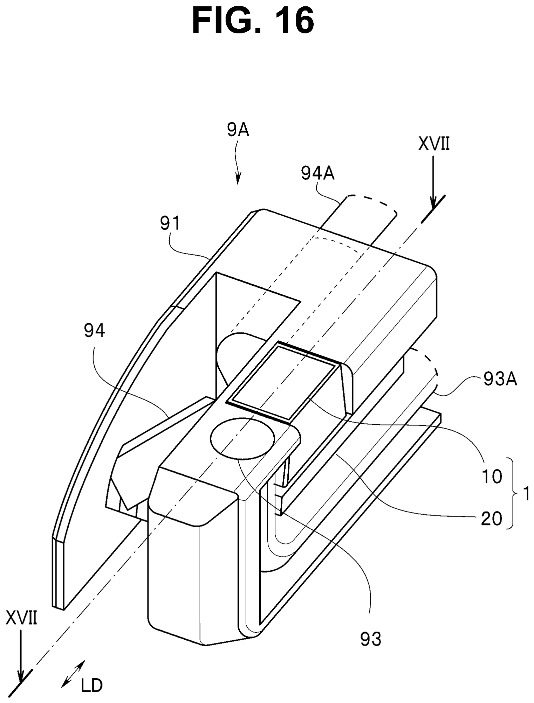

As shown in Fig. 16, in the distal end portion 9A, the image pickup module 1 is accommodated in a recess of a rigid front frame 91. In the rigid front frame 91, an illumination optical system 93, which emits illumination light transmitted from a light source apparatus (not shown) via a light guide 93A, and the image pickup module 1 are disposed.

Furthermore, a treatment instrument channel 94A is connected to the front frame 91, to enable insertion of a predetermined treatment instrument. In order to dispose the image pickup module 1 and the illumination optical system 93 at positions where the motion manner of the treatment instrument in a direction different from an insertion direction (the long axis direction LD) of the endoscope can be observed, the image pickup module 1 and the treatment instrument channel 94A need to be miniaturized.

A so-called raising base 94 for the treatment instrument is disposed in front of the treatment instrument channel 94A. The direction of a distal end of the treatment instrument, which is passed through the treatment instrument channel 94A, can be changed by the user's operation of the raising base 94. An endoscope that is further miniaturized relative to the endoscope 9 can also be protruded from the raising base 94.

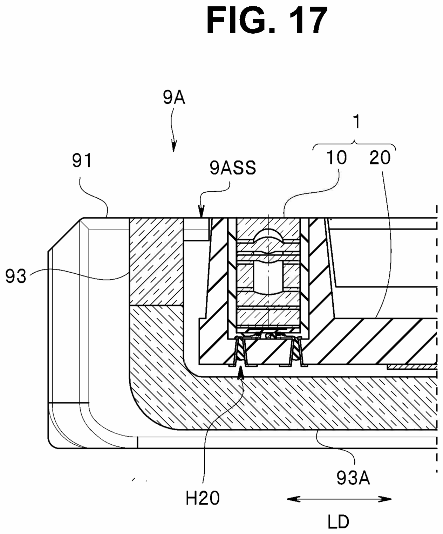

As shown in Fig. 17, in the side-viewing type endoscope 9, the plurality of through holes H20 of the image pickup module 1 extend in a direction intersecting the long axis direction LD of the distal end portion 9A. The inner dimension of each of the plurality of through holes H20 increases in a direction from an outer surface 9ASS of the distal end portion 9A, in which the image pickup module 1 is disposed, toward an interior of the distal end portion 9A.

The endoscope 9 includes the image pickup module 1, thereby achieving high performance.

It is noted that the endoscope 9 is a flexible medical endoscope, but an endoscope of another embodiment may be an industrial endoscope or a rigid endoscope including a rigid straight pipe instead of the flexible portion 9C. The image pickup module 1 may also be used in a direct-viewing type endoscope in which the object direction is in the distal end direction.

The three-dimensional circuit device is not limited to a molded interconnect device. For example, a three-dimensional circuit device may be fabricated by a machining or cutting process with a 3D printer. The material of the three-dimensional circuit device is also not limited to resin, but ceramic or glass epoxy may be used.

The present disclosure is not limited to the above-mentioned embodiments, etc., and various changes, modifications, etc. can be made without departing from the gist of the present disclosure.

### DESCRIPTION OF THE REFERENCE NUMERALS

- 1: image pickup module
- 9: endoscope
- 9A: distal end portion
- 9B: bending portion
- 9C: flexible portion
- 9D: operation portion
- 9E: universal cord
- 10: camera unit
- 11: imager
- 12: optical element
- 13: external electrode
- 14: solder
- 14X: solder bump
- 20: wiring board
- 20A: assembly member
- 20B: protrusion
- 20X: molded substrate
- 21: land
- 22: draw-out wiring
- 23: through wiring layer
- 24: wiring pattern
- 25: pad
- 29: catalyst layer
- 30: sealing resin
- 30: resin
- 30: through wiring
- 31: solder resist patterns
- 32: sealing resin
- 33: solder resist pattern
- 40: chip capacitor
- C20: cavity
- H20: through hole

## Claims

1. An image pickup module comprising:
a wiring board including a first principal surface and a second principal surface on an opposite side of the first principal surface, a plurality of lands and a plurality of draw-out wirings extended respectively from the plurality of lands being disposed on the first principal surface, the wiring board including a plurality of through holes penetrating through the first principal surface and the second principal surface, a plurality of through wiring layers being disposed on respective inner surfaces of the plurality of through holes, the plurality of through wiring layers being in electrical communication with the plurality of draw-out wirings, respectively;
a plurality of resins disposed respectively in the plurality of through holes and each sealing an opening on a side of the first principal surface;
a plurality of bonding members bonded respectively to the plurality of lands; and
a camera unit bonded to the plurality of bonding members.

2. The image pickup module according to claim 1, wherein an end surface of each of the plurality of resins on a side of the first principal surface is located between a surface of each of the plurality of draw-out wirings and the first principal surface.

3. The image pickup module according to claim 1, wherein
a plurality of wiring patterns and a plurality of patterned solder resists are disposed on the second principal surface, the plurality of patterned solder resists being for mounting an electronic component on each of the plurality of wiring patterns, and
the plurality of resins is the same as the plurality of patterned solder resists.

4. The image pickup module according to claim 1, wherein
the wiring board is a three-dimensional wiring board including a bottom surface and a protrusion, the first principal surface serving as the bottom surface, the protrusion being surrounded by walls and including a cavity, and
the camera unit is housed in the cavity.

5. The image pickup module according to claim 4, wherein
the wiring board is a molded interconnect device, and
a distance between an inner wall surface of the cavity and an end portion of each of the plurality of draw-out wirings is greater than 0.2 mm.

6. The image pickup module according to claim 1, wherein
the bonding member is solder,
the plurality of lands includes a first land and a plurality of second lands, the plurality of second lands being placed at N symmetrical positions (N being a natural number equal to or greater than 2) around the first land, and
areas of regions where the solder spreads on the plurality of draw-out wirings are substantially the same.

7. The image pickup module according to claim 6, further comprising:
a dummy draw-out wiring extended from the first land and connected to one of the second lands, both end portions of the dummy draw-out wiring being covered by solder resist patterns, respectively.

8. The image pickup module according to claim 1, wherein
an inner dimension of each of the plurality of through holes increases from the first principal surface to the second principal surface, and
an inner surface of each of the plurality of through wiring has a step.

9. The image pickup module according to claim 1, wherein the camera unit includes a plurality of optical elements and an imager stacked together.

10. An endoscope including an insertion portion including a distal end portion in which an image pickup module is disposed, the image pickup module comprising:
a wiring board including a first principal surface and a second principal surface on an opposite side of the first principal surface, a plurality of lands and a plurality of draw-out wirings extended respectively from the plurality of lands being disposed on the first principal surface, the wiring board including a plurality of through holes penetrating through the first principal surface and the second principal surface, a plurality of through wiring layers being disposed on respective inner surfaces of the plurality of through holes, the plurality of through wiring layers being in electrical communication with the plurality of draw-out wirings, respectively;
a plurality of resins disposed respectively in the plurality of through holes and each sealing an opening on a side of the first principal surface;
a plurality of bonding members bonded respectively to the plurality of lands; and
a camera unit bonded to the plurality of bonding members.

11. The endoscope according to claim 10, wherein the plurality of through holes extend in a direction intersecting a long axis direction of the distal end portion.

12. The endoscope according to claim 10, wherein an inner dimension of each of the plurality of through holes increases in a direction from an outer surface of the distal end portion, on which the image pickup module is disposed, toward an interior of the distal end portion.

13. A method for manufacturing an image pickup module, the method comprising:
fabricating a wiring board including a first principal surface and a second principal surface on an opposite side of the first principal surface, a plurality of lands and a plurality of draw-out wirings extended respectively from the plurality of lands being disposed on the first principal surface, the wiring board including a plurality of through holes penetrating through the first principal surface and the second principal surface, a plurality of through wiring layers being disposed on respective inner surfaces of the plurality of through holes, the plurality of through wiring layers being in electrical communication with the plurality of draw-out wirings, respectively;
disposing a plurality of resins in the plurality of through holes, respectively, to thereby sealing an opening of each of the plurality of through wiring layers on a side of the first principal surface; and
bonding a plurality of external electrodes of a camera unit to the plurality of lands, respectively, by using a bonding member.

14. The method for manufacturing the image pickup module according to claim 13, further comprising:
in fabricating the wiring board,
disposing the plurality of lands, the plurality of draw-out wirings, and the plurality of through wiring layers, and simultaneously disposing a plurality of wiring patterns on the second principal surface, the plurality of wiring patterns being in electrical communication with the plurality of through wiring layers, respectively;
disposing a plurality of patterned solder resists on the plurality of wiring patterns to mount an electronic component on each of the plurality of wiring patterns; and
mounting the electronic component on each of the plurality of wiring patterns, wherein
the plurality of resins is disposed simultaneously with the solder resists, the plurality of resins being the same as the solder resists.

15. The method for manufacturing the image pickup module according to claim 13, further comprising:
to fabricate the wiring board,
molding a three-dimensional substrate;
forming a through hole and an active layer by laser irradiation; and
disposing, by a plating method, the plurality of lands, the plurality of draw-out wirings, and the plurality of through wiring layers.
